# EUROPEAN PATENT SPECIFICATION

(11) **EP 2 139 562 B1**
(45) Date of publication and mention of the grant of the patent: **03.07.2013**
(21) Application number: 08717229.2
(22) Date of filing: 28.02.2008
(51) Int. Cl.: A61Q 11/00, A61K 8/46, A61K 8/73, A61K 8/92

(54) **TOOTHPASTE COMPOSITION**
ZAHNPASTAZUSAMMENSETZUNG
COMPOSITION DE PÂTE DENTIFRICE

(30) Priority: 02.04.2007 EP 07105493
(43) Date of publication of application: 06.01.2010
(73) Proprietor: Unilever PLC, A Company Registered in England and Wales under Company no. 41424, London EC4Y 0DY Greater London (GB); Unilever N.V., 3013 AL Rotterdam (NL)
(72) Inventor: FARAVELLI, Ilaria, I-26841 Casalpusterlengo (Lo), Lodi (IT); GREGORY, Donald, Peter, Bebington, Wirral Merseyside CH63 3JW (GB); INGEGNERI, Irene, I-26841 Casalpusterlengo (lo), Lodi (IT)
(74) Representative: Tansley, Sally Elizabeth
(86) International application number: PCT/EP2008/052445
(87) International publication number: WO 2008/119605

(56) References cited:
- EP-A- 0 427 210
- EP-A- 1 437 049
- EP-A- 1 726 288
- EP-A1- 1 053 743
- WO-A-01/52822
- WO-A-99/44572
- WO-A-99/59535
- GB-A- 2 132 996
- US-A- 4 565 692
- DATABASE WPI Week 200719 Derwent Publications Ltd., London, GB; AN 2007-188169 XP002450328 & JP 2007 045743 A 22 February 2007 (2007-02-22)

## Description

The present invention relates to toothpaste compositions having superior in use sensory benefits.

There are very many toothpaste compositions known in the art. Some of these comprise oils, such a flavouring oils or triglyceride oils. We have found that such compositions can have in use sensory problems because of the effect of the oil. In particular, we have found that the nature of the foam generated by the surfactant present in the toothpaste can be adversely affected.

Surprisingly, we have found that superior sensory benefits may be attained via the incorporation of a sulphated or sulphonated polysaccharide and a carboxylated polysaccharide into a toothpaste composition comprising sunflower oil. In use sensory benefits, in particular the perceived foam quality of the product, are significantly improved by the present invention.

In a first aspect of the present invention, there is provided a toothpaste composition comprising a surfactant, an Abrasive, a humectant, sunflower oil, a carboxylated polysaccharide, and sulphated or sulphonated polysaccharide, in which the amount of sunflower oil is from 0-5% to less than 10% by weight of the total composition. In a second of aspect of the present invention, there is provided a method of cleaning the teeth comprising brushing with a composition according to the first aspect of the invention.

In a third aspect of the present invention, there is provided the use of a sulphated or sulphonated polysaccharide, in particular a carrageenan, to improve the in use foam quality of a toothpaste also comprising a surfactant, an abrasive, a humectant, sunflower oil, and a carboxylated polysaccharide, the amount of sunflower oil being from 0-5% to less than 10% by weight of the total composition.

The surfactant used in accordance with the invention may be an anionic, nonionic, cationic, zwitterionic or amphoteric surfactant, or mixtures thereof. Preferably, the surfactant is anionic, in particular an alkali metal alkyl sulphate, and especially sodium lauryl sulphate (SLS).

The total amount of surfactant is typically from 0.1 to 10%, in particular from 0.5 to 5%, and especially from 1 to 3% by weight of the total composition.

The abrasive used in accordance with the invention is typically a particulate abrasive material such as a silica, alumina, calcium carbonate, dicalciumphosphate, calcium pyrophosphate, hydroxyapatite, trimetaphosphate, insoluble hexametaphosphate, or mixtures thereof. Agglomerated particulate abrasive materials may also be used. Preferred abrasive materials are chalk and silica, in particular silica.

The total amount of abrasive is typically from 3 to 60%, in particular from 5 to 40%, and especially from 6% to 20% by weight of the total composition.

The humectant, used in accordance with the invention is typically a polyol, in particular a polyol selected from glycerol, sorbitol, propyleneglycol, xylitol, lactitol, or mixtures thereof. Sorbitol is especially preferred, this material also functioning as a sweetener (*vide infra*).

The total amount of humectant is typically from 1 to 60%, in particular from 5 to 50%, and especially from 10% to 30% by weight of the total composition.

Sunflower oil is used in accordance with the invention, said oil providing a valuable source of Vitamin F to the teeth. With compositions comprising sunflower oil, the sensory benefits of the present invention have been particularly evident.

The total amount of sunflower oil is from 0.5%, in particular from 1%, and especially from 1.5% by weight of the total composition. In order that the sensory benefits associated with the sunflower oil are not too difficult to overcome, the amount of sunflower oil is less than 10% in particular less 7%, and especially less than 5% by weight of the total composition.

The carboxylated polysaccharide used in accordance with the invention is typically a cellulose derivative, in particular a carboxyalkyl cellulose, and especially carboxymethylcellulose (CMC), typically used as its sodium salt (SCMC). The carboxylated polysaccharide typically serves to thicken the toothpaste and also contributes to the sensory properties of the toothpaste.

The total amount of carboxylated polysaccharide is typically from 0.2%, in particular from 0.5%, and especially from 0.8% by weight of the total composition. The total amount is preferably less than 5%, more preferably less 2%, and most preferably less than 1% by weight of the total composition.

The sulphated or sulphonated polysaccharide used in accordance with the invention is typically a sulphated polysaccharide, in particular a linear sulphated polysaccharide, and especially a carrageenan. Of the three main commercial classes of carrageenan (Kappa, ex *Kappaphycus cottonii;* Iota, ex *Eucheuma spinosum;* and Lambda, the common source of which is *Gigartina);* Kappa and Iota are preferred. The sulphated or sulphonated polysaccharide typically serves to thicken the toothpaste and also contributes beneficially to the sensory properties of the toothpaste.

The total amount of sulphated or sulphonated polysaccharide is preferably from 0.1%, more preferably from 0.25%, and most preferably from 0.4% by weight of the total composition. The total amount is preferably less than 3%, more preferably less 1.5%, and most preferably less than 0.6% by weight of the total composition.

The weight ratio of the total amount of carboxylated polysaccharide to the total amount of sulphated or sulphonated polysaccharide is preferably from 1:2 to 10:1, more preferably from 1:1 to 5:1, most preferably from 3:2 to 3:1.

The preferable, more preferable, and most preferable total amounts of sulphated or sulphonated polysaccharide and the preferable, more preferable, and most preferable ratios of carboxylated polysaccharide to sulphated or sulphonated polysaccharide are believed to be particularly beneficial in delivering the sensory benefits of the present invention, especially at the more typical levels of surfactant and oil inclusion.

The toothpaste composition according to the invention may comprise further ingredients. In addition to the aforementioned cellulose derivatives, other materials that modify the rheological properties of the toothpaste may also be employed. Preferred additional ingredients of this type are polyethylene glycols and thickening silicas, such as Tixosil 43^{®}, ex Rhodia. Other ingredients of this type are xanthan gum, gum Arabic, synthetic polyacrylates, and synthetic carboxyvinyl polymers such as carbopol^{®}.

In addition to the aforementioned sunflower oil, other oils such as flavouring oils may also be present. Typical flavouring oils include peppermint and spearmint oil.

Vitamins sources, in particular sources of vitamin E, such as vitamin E acetate are a preferred further ingredient. A source of vitamin E is particular preferred in compositions also comprising sunflower oil, so that sources of both vitamin E and vitamin F are present (*vide supra*).

Antimicrobial agents are further ingredients that may be advantageously employed in compositions of the invention.

Examples include triclosan, chlorhexidine, copper, zinc and stannous salts (such as zinc citrate, zinc sulphate, zinc glycinate, sodium zinc citrate, stannous pyrophosphate), sanguinarine extract, metronidazole, quaternary ammonium compounds (such as cetylpyridinium chloride), bis-guanides (such as chlorhexidine), digluconate, hexetidine, octenidine, alexidine; and halogenated bisphenolic compounds (such as 2,2'-methylenebis-(4-chloro-6-bromophenol)).

Anti-caries agents are further ingredients that may be advantageously employed in compositions of the invention. Examples of such agents include sodium and stannous fluoride, aminefluorides, sodium monofluorophosphate, sodium trimetaphosphate and casein.

Polymeric compounds which can enhance the delivery of active ingredients, such as antimicrobial agents, can also be included. Examples of such polymers are copolymers of polyvinylmethylether with maleic anhydride and other similar delivery enhancing polymers, e.g. those described in DE-A-3,942,643 (Colgate).

Other ingredients that may be incorporated include:
plaque buffers, such as urea, calcium lactate, calcium glycerophosphate and strontium polyacrylates;
anti-inflammatory agents, such as ibuprofen, flurbiprofen, aspirin, and indomethacin;
desensitising agents, such as potassium citrate, potassium chloride, potassium tartrate, potassium bicarbonate, potassium oxalate, potassium nitrate and strontium salts;
anti-calculus agents, such as alkali-metal pyrophosphates, hypophosphite-containing polymers, organic phosphonates and phosphocitrates;
bio-molecules, such as bacteriocins, antibodies, and enzymes;
proteinaceous materials, such as collagen;
preservatives;
opacifying agents;
colouring agents;
pH-adjusting agents;
sweetening agents, such as saccharin and sorbitol *(vide supra);*
bleaching agents, such as peroxy compounds (e.g. potassium peroxydiphosphate);
buffers and salts to buffer the pH and ionic strength of the composition; and
pharmaceutically acceptable carriers, e.g. starch, sucrose, water or water/alcohol systems. Water is a particularly preferred pharmaceutically acceptable carrier and is typically present in compositions of the invention at from 5 to 95%, in particular 15 to 75%, and especially at from 35 to 60% by weight of the total composition.

### Examples

In the following examples, comparative examples are indicated by letters and examples according to the invention are indicated by numbers. All amounts of components are indicated as percentages by weight.

The toothpaste compositions indicated in Table 1 were prepared by standard methods known in the art. The compositions are identical, with the exception that 0.5% of the SCMC in comparative example A is replaced by carrangeenan in example 1.

**Table 1**

| **Component** | **Trade name** | **Supplier** | **Example** | |
|---|---|---|---|---|
| | | | **A** | **1** |
| Sorbitol soln. | Neosorb 70/70 | Cerestar | 25.00 | 25.00 |
| Abrasive silica | Sorbosil AC77 | Ineos | 9.00 | 9.00 |
| Thickening silica | Tixosil 43 | Rhodia | 7.50 | 7.50 |
| Poly(ethylene glycol) | PEG 32 | BASF | 5.00 | 5.00 |
| Surfactant | SLS | Unger | 1.80 | 1.80 |
| Flavouring oil | | | 1.50 | 1.50 |
| Zinc citrate | | Tate&Lyle | 0.75 | 0.75 |
| Sunflower oil | Akosun-bha-F¹ | Akosun | 0.50 | 0.50 |
| SCMC | CMC 9M | Cekol | 1.40 | 0.90 |
| Carrageenan | C'geenan TPC1 | Cp Kelko | -- | 0.50 |
| Phenoxyethanol | | Lanxess | 0.40 | 0.40 |
| Sodium fluoride | | Honeywell | 0.32 | 0.32 |
| Anti-microbial | Triclosan | Ciba | 0.30 | 0.30 |
| Water + minors | | | To 100 | To 100 |

A performance evaluation test of the compositions indicated in Table 1 was carried in order to evaluate user perceptions of the products. 21 trained panellists were employed and asked to evaluate the toothpastes across a range of attributes using a 0 to 10 line scale. The average results concerning the in use perceptions of the toothpastes are indicated in Table 2.

**Table 2**

| **Attribute** | **Score** | |
|---|---|---|
| | **Example A** | **Example 1** |
| | | |
| Grittiness | 0.6 | 0.5 |
| Foam quantity | 6.4 | 6.4 |
| Foam quality | 6.3 | 6.8 |

The composition in accordance with the invention was found to give a significantly higher foam quality perception than the comparative example.

## Claims

1. A toothpaste composition comprising a surfactant, an abrasive, a humectant, sunflower oil, a carboxylated polysaccharide, and a sulphated or sulphonated polysaccharide, the amount of sunflower oil being from 0.5% to less than 10% by weight of the total composition.

2. A composition according to claim 1, wherein the surfactant is an anionic surfactant.

3. A composition according to claim 1 or claim 2, wherein the surfactant is sodium lauryl sulphate.

4. A composition according to any of the preceding claims, further comprising a flavouring oil.

5. A composition according to any of the preceding claims, wherein the carboxylated polysaccharide is a carboxymethylcellulose.

6. A composition according to any of the preceding claims, wherein the sulphated or sulphonated polysaccharide is a carrageenan.

7. A composition according to any of the preceding claims, wherein the sunflower oil is present at a level of from 0.5% to less than 10%, the carboxylated polysaccharide is present at a level of from 0.5%, and the sulphated or sulphonated polysaccharide is present at a level of from 0.25%, all percentages being by weight.

8. A composition according to any Of the preceding claims, wherein the total amount of carboxylated polysaccharide to the total amount of sulphated or sulphonated polysaccharide is from 3:2 to 3:1.

9. A method of cleaning the teeth comprising brushing with a composition according to any of claims 1 to

10. The use of a sulphated or sulphonated polysaccharide, in particular a carrageenan, to improve the in use foam quality of a toothpaste also comprising a surfactant, an abrasive, a humectant, sunflower oil, and a carboxylated polysaccharide, the amount of sunflower oil being from 0.5% to less than 10% by weight of the total composition.

## Patentansprüche

1. Zahnpastazusammensetzung, die ein Tensid, ein Abrasivmittel, ein Feuchthaltemittel, Sonnenblumenöl, ein carboxyliertes Polysaccharid und ein sulfatiertes oder sulfoniertes Polysaccharid umfasst, wobei die Menge an Sonnenblumenöl 0,5 bis weniger als 10 Gew.-% der gesamten Zusammensetzung ist.

2. Zusammensetzung gemäß Anspruch 1, wobei das Tensid ein anionisches Tensid ist.

3. Zusammensetzung gemäß Anspruch 1 oder Anspruch 2, wobei das Tensid Natriumlaurylsulfat ist.

4. Zusammensetzung gemäß einem der vorangehenden Ansprüche, die außerdem ein aromatisierendes Öl umfasst.

5. Zusammensetzung gemäß einem der vorangehenden Ansprüche, wobei das carboxylierte Polysaccharid eine Carboxymethylcellulose ist.

6. Zusammensetzung gemäß einem der vorangehenden Ansprüche, wobei das sulfatierte oder sulfonierte Polysaccharid Carrageenan ist.

7. Zusammensetzung gemäß einem der vorangehenden Ansprüche, wobei das Sonnenblumenöl in einer Konzentration von 0,5 bis weniger als 10 % vorliegt, das carboxylierte Polysaccharid in einer Konzentration von 0,5 % vorliegt und das sulfatierte oder sulfonierte Polysaccharid in einer Konzentration von 0,25 % vorliegt, wobei alle Prozentangaben Gewichtsprozente sind.

8. Zusammensetzung gemäß einem der vorangehenden Ansprüche, wobei die gesamte Menge an carboxyliertem Polysaccharid zu der gesamten Menge an sulfatiertem oder sulfoniertem Polysaccharid 3:2 bis 3:1 ist.

9. Verfahren zur Reinigung der Zähne, das Bürsten mit einer Zusammensetzung gemäß einem der Ansprüche 1 bis 8 umfasst.

10. Verwendung eines sulfatierten oder sulfonierten Polysaccharids, insbesondere eines Carrageenans, um die Schaumqualität bei der Verwendung einer Zahnpasta zu verbessern, auch umfassend ein Tensid, ein Abrasivmittel, ein Feuchthaltemittel, Sonnenblumenöl und ein carboxyliertes Polysaccharid, wobei die Menge an Sonnenblumenöl 0,5 bis weniger als 10 Gew.-% der gesamten Zusammensetzung ist.

## Revendications

1. Composition de pâte dentifrice comprenant un tensioactif, un abrasif, un humectant, de l'huile de tournesol, un polysaccharide carboxylé, et un polysaccharide sulfaté ou sulfoné, la quantité d'huile de tournesol étant de 0,5 à moins de 10 % en poids de la composition totale.

2. Composition selon la revendication 1, dans laquelle le tensioactif est un tensioactif anionique.

3. Composition selon la revendication 1 ou la revendication 2, dans laquelle le tensioactif est le laurylsulfate de sodium.

4. Composition selon l'une quelconque des revendications précédentes comprenant en outre une huile aromatisante.

5. Composition selon l'une quelconque des revendications précédentes, dans laquelle le polysaccharide carboxylé est la carboxyméthyl-cellulose.

6. Composition selon l'une quelconque des revendications précédentes, dans laquelle le polysaccharide sulfaté ou sulfoné est une carraghénine.

7. Composition selon l'une quelconque des revendications précédentes, dans laquelle l'huile de tournesol est présente à une teneur depuis 0,5 à moins de 10 %, le polysaccharide carboxylé est présent à une teneur depuis 0,5 %, et le polysaccharide sulfaté ou sulfoné est présent une teneur depuis 0,25 %, tous les pourcentages étant en poids.

8. Composition selon l'une quelconque des revendications précédentes, dans laquelle la quantité totale de polysaccharide carboxylé à la quantité totale de polysaccharide sulfaté ou sulfoné est de 3:2 à 3:1.

9. Procédé de nettoyage des dents comprenant le brossage avec une composition selon l'une quelconque des revendications 1 à 8.

10. Utilisation d'un polysaccharide sulfaté ou sulfoné, en particulier, d'une carraghénine, pour améliorer la qualité de mousse d'une pâte dentifrice en utilisation comprenant également un tensioactif, un abrasif, un humectant, de l'huile de tournesol, et un polysaccharide carboxylé, la quantité d'huile de tournesol étant de 0,5 à moins de 10 % en poids de la composition totale.
